(19) Europäisches Patentamt / European Patent Office / Office européen des brevets

(11) **EP 1 070 954 B1**

(12) **FASCICULE DE BREVET EUROPEEN**

(45) Date de publication et mention
de la délivrance du brevet:
**05.11.2008 Bulletin 2008/45**

(51) Int Cl.:
*G01N 21/33* *(2006.01)*     *G01N 33/18* *(2006.01)*
*G01N 21/27* *(2006.01)*

(21) Numéro de dépôt: **00402038.4**

(22) Date de dépôt: **17.07.2000**

(54) **Procédé et dispositif pour l'analyse d'un échantillon liquide.**

Verfahren und Vorrichtung zur Analyse einer flüssigen Probe

Method and device for analysis of a liquid sample

(84) Etats contractants désignés:
**BE CH DE GB LI**

(30) Priorité: **20.07.1999 FR 9909573**

(43) Date de publication de la demande:
**24.01.2001 Bulletin 2001/04**

(73) Titulaire: **Secomam**
**95334 Domont Cedex (FR)**

(72) Inventeurs:
• **Constant, Daniel**
**95330 Domont (FR)**
• **Rigal, Jean-Marc**
**95330 Domont (FR)**

(74) Mandataire: **de Saint-Palais, Arnaud Marie**
**CABINET MOUTARD**
**35, rue de la Paroisse**
**B.P. 513**
**78005 Versailles Cedex (FR)**

(56) Documents cités:
EP-A- 0 682 250     EP-A- 0 838 676
US-A- 4 304 996     US-A- 4 660 151
US-A- 5 528 368     US-A- 5 886 347

• **O.THOMAS ET AL: "Advanced uv examintaion of wastewater" ENVIRONMENTAL TECHNOLOGY, vol. 17, 1996, pages 251-261, XP000856285**
• **THOMAS O ET AL: "UV SPECTRAL DECONVOLUTION: A VALUABLE TOOL FOR WASTE WATER QUALITY DETERMINATION" ENVIRONMENTAL TECHNOLOGY, vol. 14, septembre 1993 (1993-09), pages 1187-1192, XP000856278**

**Description**

La présente invention concerne un procédé et un dispositif pour l'analyse d'un échantillon liquide.

Elle s'applique notamment, mais non exclusivement, au contrôle de qualité et à la détection de pollutions pouvant affecter des effluents liquides tels que, par exemple, ceux rejetés par des sites urbains ou industriels.

Par la publication de O. Thomas et al, "Advanced UV examination of wastewater", ENVIRONMENTAL TECHNOLOGY, Vol. 17, 1996, pages 251-261, on a proposé un procédé d'analyse d'un effluent liquide par spectrophotométrie UV utilisant une base standard des spectres de référence, comprenant cinq spectres correspondant aux trois différentes phases d'une matière absorbante (suspendue, colloïde et dissoute) et à deux composés spécifiques (nitrates et détergents).

Il s'avère que les causes de pollution sont trop nombreuses pour que l'on puisse les détecter instantanément par des analyses périodiques des effluents: seules les pollutions les plus courantes peuvent être prises en compte. Par contre, lorsqu'il s'agit d'une cause de pollution rare et/ou imprévisible, la pollution n'est décelée que bien après sa naissance, à la suite d'un constat de ses conséquences sur l'environnement. Dans certains cas, la pollution est détectée bien après que sa cause première ait disparue.

En vue de résoudre ce problème, l'invention propose d'exercer une surveillance dans le temps de l'effluent de manière à pouvoir être en mesure, lorsqu'une pollution a été détectée, de déterminer le moment précis où cette pollution a commencée à se manifester et d'étudier l'évolution dans le temps de cette pollution jusqu'au moment de sa détection et même au-delà. L'objectif est alors de pouvoir disposer d'un maximum d'informations pour trouver la cause de la pollution et, éventuellement, de découvrir ses responsables.

L'invention parvient à ces résultats grâce à un procédé comportant une succession de cycles opératoires répétés périodiquement et comprenant les étapes spécifiées dans la revendication 1.

Grâce à ces dispositions, lorsqu'une pollution a été détecté, il est possible d'effectuer un traitement numérique des données correspondant à chacun de ces prélèvements pour détecter la présence de la substance polluante et donc de connaître très précisément le moment où cette substance est apparue pour la première fois dans l'effluent, et les éventuelles variations dans le temps de sa teneur au sein de l'effluent.

Pour obtenir ces informations, l'invention propose de mettre en oeuvre un procédé et un dispositif de caractérisation de matières dissoutes ou en suspension dans un liquide du type de ceux décrits dans la demande de brevet européen No 0 682 250.

On sait que ce procédé permet d'effectuer cette caractérisation par absorptiométrie ultraviolette en exploitant le spectre d'absorption de l'eau pour en déduire les valeurs de certains paramètres utiles en vue de déterminer les concentrations des éléments ou composés recherchés et d'autres données physico-chimiques caractéristiques de l'échantillon.

Ainsi, pour estimer la concentration des molécules contenant du carbone organique ou les matières en suspension, on a proposé de déterminer, à l'aide d'un spectrophotomètre, la variation de l'absorbance dans une plage de longueurs d'ondes comprise entre 200 et 300 nanomètres.

Dans ce cas, la relation entre le signal UV et les molécules contenant du carbone organique (paramètre le plus significatif de la présence des matières organiques), s'appuie sur la relation de Beer Lambert dont la propriété le d'additivité permet de dire que l'absorbance mesurée, dans le cas d'un mélange en solution diluée, est égale à la somme des absorbances de chaque constituant dans la solution. Ces absorbances sont fonction de la concentration en carbone organique puisque cette dernière est liée à la concentration du constituant.

Cependant, selon la nature des molécules organiques prédominantes dans l'échantillon qui varie avec le type d'effluent, la relation entre le carbone organique et le spectre sera différente puisqu'une même quantité de carbone peut être associée à un nombre différent de chromophores.

Pour tenir compte de ce facteur, on a donc exploité le fait que le spectre d'un échantillon inconnu peut être considéré comme une combinaison linéaire d'un petit nombre de spectres correspondant à des situations de référence parfaitement caractérisées. Ces spectres de référence SR, qui sont caractéristiques des états de la matière organique, permettent de définir tout spectre inconnu SI sous la forme d'une combinaison linéaire:

$$SI = a_1\ SR_1 + a_2\ SR_2 + \text{-------} + a_p\ SR_P$$

où $a_1$ ------- $a_p$ sont les coefficients des p spectres de référence $SR_1$ ------- $SR_P$.

La connaissance des spectres de référence et de leurs coefficients, et des caractéristiques physico-chimiques des échantillons dont ils proviennent, permet de calculer n'importe quel paramètre absorbant dans l'Ultra Violet de l'échantillon inconnu (en supposant que ce paramètre soit additif). En particulier, la concentration en molécules contenant

du carbone organique $CO_i$ d'un échantillon inconnu est calculée à l'aide de la même combinaison linéaire, appliquée cependant aux concentrations respectives de molécules contenant du carbone organique par exemple le carbone organique total COT $COT_1$ ------- $COT_P$ des spectres caractéristiques:

$$COT_i = a_1 \cdot COT_1 + a_2 \cdot COT_2 + \text{-------} \; a_p \; COT_P$$

où $COT_i$, $COT_1$, $COT_2$ ------- $COT_P$ sont des valeurs respectives de carbone organique.

Cette méthode permet donc, en utilisant un processeur programmé de manière à assurer une déconvolution des spectres avec prise en compte des interférences, de déterminer la charge organique d'une eau à partir de la contribution amenée par chacun des spectres pondérés, pour chaque échantillon.

En fait, ces spectres sont composés de ceux:

- des produits prépondérants dans la solution et absorbant dans l'Ultra Violet (par exemple les nitrates),
- composant la matrice organique générale et souvent composée de centaines de molécules différentes.

Les molécules prépondérantes sont généralement connues de l'utilisateur. Leur choix est, soit fixé au préalable, soit extrait d'une bibliothèque suivant des critères tels que par exemple ceux qui sont définis dans les demandes de brevet FR No 2783322 et FR No 2787883 au nom de la Société Naphtachimie.

Cette méthode permet donc, en particulier, de déterminer, à partir des informations relatives aux spectres détectés par un détecteur, la teneur en carbone organique, en azote nitreux et nitrique, en matières en suspension d'un liquide, par exemple d'un effluent, soumis à un phénomène de photooxydation pour révéler des substances non décelables directement à partir de spectre UV. Le processeur pourra en outre effectuer un calcul des formes azotées oxydables et totales, des phosphates ainsi que l'oxydabilité en mesurant, par exemple, le temps de stabilisation des mesures d'un échantillon circulant dans le circuit de photooxydation en boucle fermée.

Toutefois, il s'avère que jusqu'ici, le calcul des spectres de la matrice est jusqu'à présent déterminé manuellement de façon grossière par analogie avec des échantillons existants. Il en est de même pour la contribution à la concentration de l'échantillon.

En effet, du fait que le spectre de l'échantillon à analyser est la somme des spectres d'un grand nombre de molécules, il est quasiment impossible de prédire sa forme à partir de cette somme.

L'invention a donc plus particulièrement pour but d'améliorer le procédé précédemment défini en procédant à la modélisation du spectre à la fois à partir de spectres de produits purs identifiés préalablement mémorisés et à partir d'échantillons du liquide à mesurer prélevés dans des conditions différentes.

Elle propose un procédé pour la surveillance dans le temps d'un effluent liquide qui comporte une succession de cycles opératoires du type de celle définie dans la publication de O.THOMAS précitée.

Ce procédé est par ailleurs caractérisé en ce que pour effectuer l'analyse de l'échantillon liquide, il comprend :

- le prélèvement d'un échantillon de l'effluent liquide que l'on veut surveiller,
- une analyse spectrophotométrique permettant de relever un spectre de fréquence caractéristique de ce prélèvement,
- un échantillonnage du spectre obtenu par cette analyse, de manière à obtenir une série d'échantillons du spectre à des fréquences successives,
- la numérisation de la valeur de ces échantillons de spectre,
- le stockage en mémoire, avec horodatage, des données numériques relatives à chacun de ces échantillons de spectre et des données correspondant à la fréquence de ces échantillons;

une phase préalable comportant :

- l'acquisition de spectres échantillons de référence obtenus par spectrométrie UV d'une pluralité d'échantillons de référence différents, dudit effluant liquide, la mesure de ces spectres échantillon de référence et la détermination en laboratoire des paramètres de ces échantillons de référence, par des méthodes de référence
- la constitution d'une première bibliothèque incluant des données relatives à ces spectres échantillons de référence
- l'acquisition et le stockage dans une seconde bibliothèque des données relatives à des spectres d'absorption UV de produis purs connus entrant dans la composition dudit effluent liquide
- la constitution d'une base de modélisation contenant les données relatives à un nombre minimum N de spectres dont la combinaison linéaire permet d'obtenir un spectre modélisé représentatif du spectre de l'échantillon à analyser, ces données comprenant d'une part, un petit nombre M (M < N) de spectres sélectionnés de produits purs connus dont les données sont stockées dans la seconde bibliothèque et un nombre N-M de spectres calculés à partir de spectres sélectionnés et de spectres échantillons;

- l'obtention d'un modèle de spectre obtenu par combinaison linéaire des spectres de la base de modélisation, et

une phase opératoire comportant,

- la comparaison du modèle de spectre et du spectre d'absorption de l'échantillon inconnu à analyser de manière à vérifier la pertinence du modèle.
- la détermination, par un calcul de déconvolution, des coefficients de pondération des spectres de la base de modélisation dans la quantification du spectre d'absorption de l'échantillon à analyser,
- la calibration du modèle par le calcul des paramètres de l'effluent liquide en fonction de la valeur des susdits coefficients de pondération,

la constitution de la base de modélisation étant obtenue à l'issue d'un processus incrémental comprenant les phases suivantes:

- la sélection et l'introduction dans la base de modélisation des données relatives aux M spectres de produits purs connus,
- la sélection d'un spectre échantillon « remarquable » contenu dans la première bibliothèque en effectuant une déconvolution de chaque spectre échantillon pour connaître la contribution des produits purs connus dans ce spectre échantillon et pour déterminer la fraction résiduelle de ce spectre échantillon par rapport aux spectres connus en choisissant le spectre échantillon « remarquable » dont la fraction résiduelle est la plus importante,
- l'extraction de la partie du spectre sélectionné qui n'est pas présente dans les spectres déjà rentrés en retranchant au spectre sélectionné une fraction (par exemple 45 %) de la somme des spectres déjà rentrés de manière à obtenir un nouveau spectre étalon représentatif de nouveaux produits,
- l'introduction dans la base de modélisation du nouveau spectre étalon,
- le début d'un nouveau cycle de sélection, d'extraction et d'introduction et ce, jusqu'à ce que la base de modélisation soit pleine.

Ce procédé pourra en outre comprendre la validation de cette calibration par l'établissement de la droite de régression rapprochant les résultats mesurés de ceux estimés par le calcul.

La bibliothèque de données relatives à des spectres d'absorption connus pourra comprendre des spectres de nombreux produits « étalons » susceptibles d'être présents dans le liquide à analyser.

Ainsi, dans le cas où le liquide à analyser est un effluent industriel, ces produits étalons pourront être des produits purs tels que les nitrates, le nocylène, le tributhylcathechol TBC, des ions nitrites, de l'éthylpropylacrolénie EPA, etc... ainsi que des spectres globaux d'absorption UV d'effluents qualifiés normaux.

- le début d'un nouveau cycle de sélection, d'extraction et d'introduction et ce, jusqu'à ce que la base de modélisation soit pleine. Avantageusement, à la suite de l'introduction dans la base de modélisation de chaque nouveau spectre étalon à compter du deuxième, le procédé pourra comprendre une séquence d'affinage comportant un recalcul des spectres étalons précédemment introduits dans la base en utilisant à chaque fois tous les spectres présents dans la base, autres que celui qui est recalculé.

Une fois que la base de modélisation est complète, on dispose en principe du modèle de l'échantillon liquide dont on veut mesurer les paramètres de pollution.

Toutefois, préalablement à la détermination de ces paramètres, on procède à une vérification de la pertinence du modèle présent dans la base de modélisation en s'assurant que l'écart maximum entre le spectre modélisé et les spectres échantillon demeure au-dessous d'un seuil prédéterminé.

Le calcul des paramètres de la pollution organique de l'échantillon que l'on veut mesurer comprend un calcul par déconvolution du coefficient de pondération à appliquer à chacun des spectres de la base de modélisation pour obtenir le spectre de cet échantillon, et la valeur des paramètres de chacun de ces spectres, pris séparément.

Ce calcul implique la connaissance des valeurs vraies, mesurées en laboratoire, ou par tout autre moyen des paramètres de pollution des spectres échantillon.

Il comprend, au moins pour une partie des spectres échantillon contenus dans la bibliothèque, la constitution, par un processus de déconvolution d'une matrice donnant la valeur des paramètres de pollution pour chacun des spectres de la base de modélisation, cette matrice étant de la forme indiquée ci-après dans le Tableau I ci-après.

**TABLEAU I**

| Spectres de la base de modélisation | Spectre 1 $SP_1$ | Spectre 2 $SP_2$ | Spectre 3 $SP_3$ | Spectre 4 $SP_4$ | Spectre N $SP_N$ |
|---|---|---|---|---|---|
| Paramètre 1 | $A_1$ | $A_2$ | $A_3$ | $A_4$ | $A_N$ |
| Paramètre 2 | $B_1$ | $B_2$ | $B_3$ | $B_4$ | $B_N$ |
| Paramètre 3 | $C_1$ | $C_2$ | $C_3$ | $C_4$ | $C_N$ |
| Paramètre 4 -------------- | $D_1$ -------------- | $D_2$ -------------- | $D_3$ -------------- | $D_4$ -------------- | $D_N$ -------------- |
| Paramètre P | $P_1$ | $P_2$ | $P_3$ | $P_4$ | $P_N$ |

La robustesse du calcul est alors assurée par le fait qu'on utilise un nombre de spectres échantillon en excès (par exemple deux à trois fois le nombre de spectres de la base de modélisation).

Une fois les paramètres de pollution de chacun des spectres de la base de modélisation déterminés, on procède au calcul des coefficients de contribution $C_1$, $C_2$, $C_3$ ... $C_N$ des spectres de la base de modélisation dans le spectre SPech de l'échantillon à analyser satisfaisant à la sélection

$$SPech = C_1\,SP_1 + C_2\,SP_2 + C_3\,SP_3 + \ldots.. C_N\,SP_N$$

Une fois ces coefficients déterminés, on obtient les valeurs des paramètres PX de l'échantillon à analyser à partir des relations suivantes:

$$PX_1 = C_1\,AX_1 + C_2\,AX_2 + C_3\,AX_3 + \ldots.. C_N\,AX_N$$

$$PX_2 = C_1\,BX_1 + C_2\,BX_2 + C_3\,BX_3 + \ldots.. C_N\,BX_N$$

$$PX_3 = C_1\,CX_1 + C_2\,CX_2 + C_3\,CX_3 + \ldots.. C_N\,CX_N$$

$$PX_P = C_1\,ZX_1 + C_2\,ZX_2 + C_3\,ZX_3 + \ldots.. C_N\,ZX_N$$

relations dans lesquelles $AX_1$ ..... $AX_N$ sont les valeurs du paramètre 1 dans les spectres $SP_1$ ..... $SP_N$ de la base de modélisation, et $ZX_1$ ..... $ZX_N$ sont les valeurs du paramètre P dans les spectres $SP_1$.....$SP_N$ de la base de modélisation.

L'invention concerne également un dispositif pour la mise en oeuvre du procédé précédemment décrit, ce dispositif comprenant des moyens permettant d'effectuer une photooxydation de l'échantillon pour révéler des substances dont le spectre n'apparaît pas directement aux UV, un spectrophotomètre pour l'analyse spectrophotométrique UV de l'échantillon photooxydé et un processeur programmé de manière à effectuer un échantillonnage du spectre obtenu par le spectrophotomètre, la numérisation des échantillons ainsi obtenus et la mémorisation, avec horodatage, des valeurs numériques du spectre.

Ce processeur est en outre programmé pour:

- recevoir et stocker en mémoire les données relatives à une pluralité de spectres de produits purs et à une pluralité de spectres échantillon du liquide à analyser,
- constituer une base de modélisation apte à contenir les données numériques d'un nombre N de spectres dont la combinaison linéaire permet d'obtenir un spectre modélisé représentatif du spectre de l'échantillon à analyser,

- comparer le spectre modélisé au spectre d'absorption de l'échantillon de manière à vérifier la pertinence du modèle,
- déterminer, par un calcul de déconvolution, le coefficient de pondération des spectres de la base de modélisation dans la quantification du spectre d'absorption de l'échantillon à analyser,
- calculer les paramètres de pollution en fonction de la valeur des coefficients de contribution (calibration),
- valider cette calibration par l'établissement d'une droite de régression rapprochant les résultats mesurés de ceux obtenus par le calcul.

Des modes d'exécution de dispositifs pour la mise en oeuvre du procédé selon l'invention seront décrits ci-après, à titre d'exemples non limitatifs, avec référence aux dessins annexés dans lesquels:

La figure 1 est une représentation schématique d'un appareil pour la numérisation, l'archivage et la caractérisation d'échantillons d'effluents liquides;

La figure 2 est une coupe transversale schématique de la cuve à échantillon de l'appareil représenté figure 1;

La figure 3 une représentation schématique de l'organigramme du programme exécuté par le processeur utilisé dans l'appareil de la figure 1;

La figure 4 est une représentation schématique de la base de modélisation, avec en correspondance, les valeurs des paramètres et des coefficients de pondération.

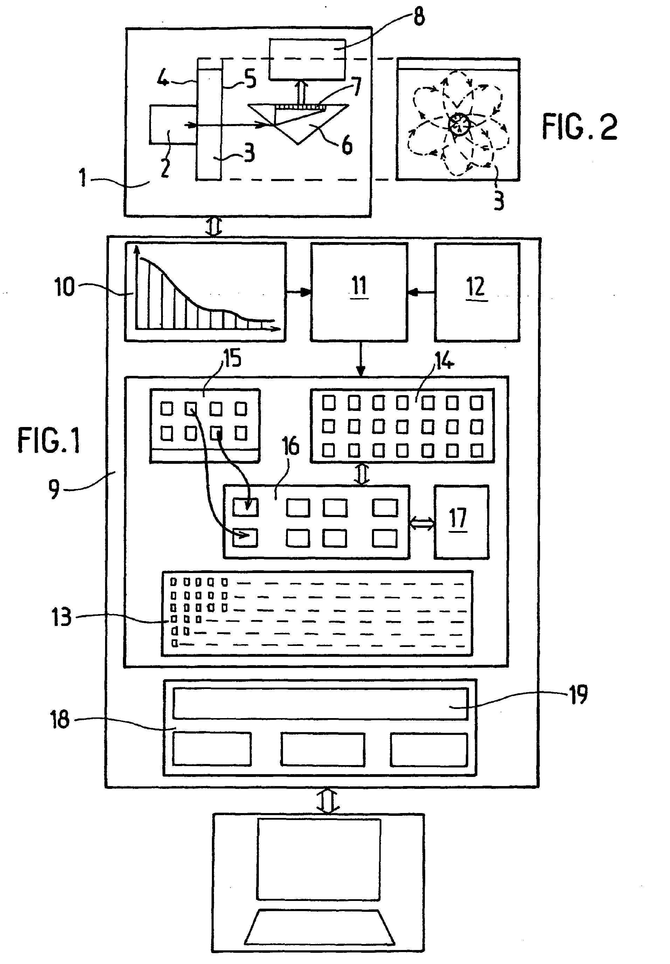

Dans l'exemple représenté sur la figure 1, l'appareil comprend tout d'abord un dispositif spectrophotométrique UV 1 faisant intervenir une source lumineuse 2 qui émet un faisceau rayonnant dans le domaine de l'ultraviolet (180 à 350 nm) en direction d'une cuve d'échantillon 3 comportant deux faces parallèles transparentes 4, 5 qui délimitent une lame d'échantillon liquide. Le faisceau qui se propage perpendiculairement aux faces 4, 5 dans la partie centrale de celle-ci traverse l'échantillon et ressort, côté opposé, à la source pour pénétrer dans la fente d'un spectromètre UV (par exemple de 205 à 400 nm) schématisé par un élément dispersif (prisme) 6 et une barrette de photodiodes 7.

Une particularité de ce dispositif consiste en ce qu'il ne comprend qu'une seule source lumineuse 2 pour effectuer à la fois la photooxydation de l'échantillon et pour obtenir sa réponse spectrale aux UV. En effet, dans les dispositifs existants, ces deux fonctions font intervenir deux sources lumineuses distinctes disposées en deux emplacements différents d'un circuit de circulation, en boucle fermée ou semi fermée de l'échantillon. Ce circuit paraissait jusqu'ici indispensable pour obtenir une photooxydation homogène et uniforme de l'échantillon au niveau souhaité.

Contrairement à ce préjugé, l'invention parvient à un résultat similaire grâce au fait qu'elle n'a à photooxyder qu'un volume réduit d'échantillon liquide et qu'elle utilise les courants de convection engendrés par l'élévation de température de la région traversée par le faisceau pour assurer l'homogénéisation de l'oxydation de l'échantillon.

La figure 2 montre les courants de convection qui se forment autour du faisceau lumineux et qui assurent une fonction analogue à celle de la circulation forcée de l'échantillon, sous l'effet d'une pompe, utilisée dans les appareils antérieurs.

Dans cet exemple, la source lumineuse 2 consiste en une lampe au deutérium à fenêtre de quartz supra sil permettant une bonne transmission des rayonnements ultraviolets lointains.

La cuve 3, également en quartz supra sil, présente un trajet optique de 2 mm contenant l'échantillon. Compte tenu de l'absorbance de l'air aux UV lointains (< 200 nm) un minimum d'air sera laissé entre la lampe et la cuve d'échantillon 3. En pratique, la lampe sera accolée à l'une des faces 4 de la cuve 3.

Les informations délivrées par les photodiodes de la barrette 7 sont transmises à un circuit électronique 8 piloté par un processeur 9 représenté schématiquement sur la figure 1 qui effectue un échantillonnage (bloc 10) des spectres détectés par la barrette 7, numérise les échantillons (bloc 11) puis les stocke en mémoire soit dans le cadre d'un processus d'archivage, soit dans le cadre d'un traitement d'analyse. Dans tous les cas, les données numériques relatives à un spectre sont associées à des données d'identification de l'échantillon qui a produit le spectre et à des données d'horodatage fournies par un horodateur (bloc 12).

Grâce à ces dispositions, il devient possible en effectuant des prélèvements périodiques d'un effluent, de stocker en archive les données spectrales de ces échantillons de manière à ce que, lorsqu'une pollution a été détectée, on puisse déterminer le moment où elle a pris naissance et connaître ensuite son évolution jusqu'à sa découverte. Il s'agit là d'informations précieuses pour identifier la pollution, déterminer sa cause ainsi que ses responsables et prendre ensuite les mesures nécessaires pour l'enrayer.

En fait, l'espace mémoire utilisé par le processeur est divisé en plusieurs zones, à savoir:

- une zone 13 destinée à assurer un archivage des données spectrales identifiées et horodatées,

- une zone bibliothèque 14 (première bibliothèque) destinée à contenir les données spectrales d'échantillons de l'effluent à surveiller et/ou à analyser et dont on connaît les principaux paramètres (également stockés en mémoire),
- une zone 15 (deuxième bibliothèque) destinée à contenir les données spectrales de produits purs susceptibles de rentrer dans la composition de l'effluent,
- une zone 16 attribuée à la base de modélisation, cette base étant destinée à contenir les données spectrales d'un nombre limité de spectres, ici huit,
- une zone 17 destinée à contenir la matrice de pondération des spectres de la base de modélisation 16.

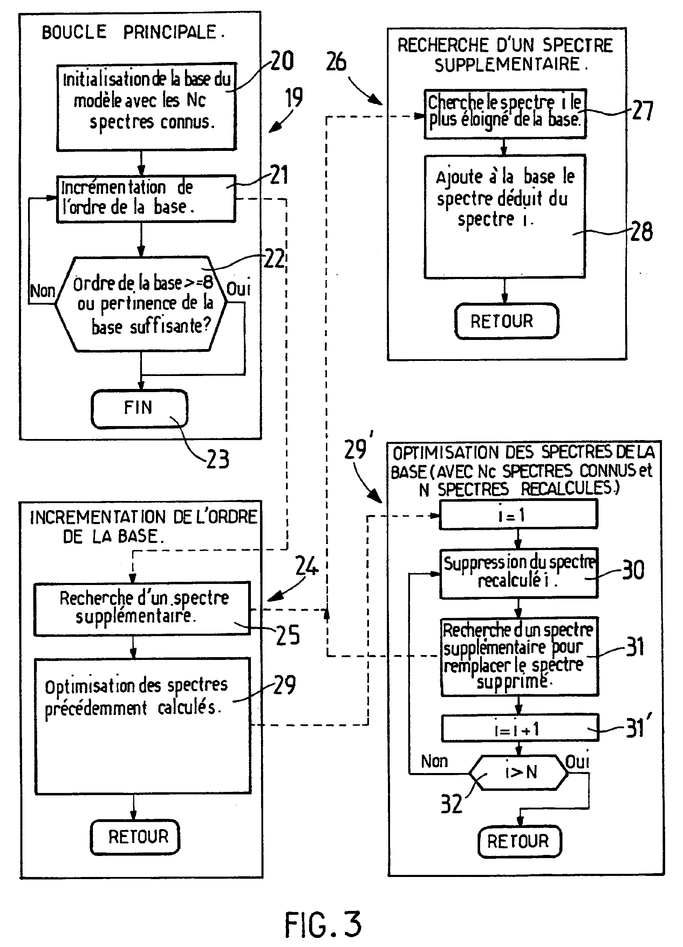

Bien entendu, l'espace mémoire comprend en outre un espace programme 18 comprenant notamment un programme de caractérisation dont l'organigramme général est illustré sur la figure 3.

Ce programme comprend une boucle principale 19 qui comporte successivement :

- l'initialisation de la base de modélisation avec des spectres connus (bloc 20),
- l'incrémentation de l'ordre de la base de modélisation (bloc 21) qui correspond au nombre de spectres déjà entrés dans la base de modélisation,
- une détection (bloc 22) pour savoir si la base est remplie et/ou si la base est pertinente,
- un rebouclage sur l'incrémentation de l'ordre de la base (bloc 21) si le résultat de la détection est négatif,
- la fin du programme (bloc 23) si ce résultat est positif.

L'incrémentation de l'ordre du modèle contenu dans la base de modélisation ne s'effectue qu'après l'implémentation d'un sous-programme 24 d'incrémentation de l'ordre du modèle comportant:

- une étape de recherche d'un spectre supplémentaire implémentée (bloc 25) par un sous-programme 26 qui recherche le spectre échantillon le plus éloigné de la base (bloc 27) de modélisation et qui ajoute (bloc 28) à cette base de modélisation un spectre déduit du spectre i (spectre i auquel on a retranché une portion du spectre calculé par la base précédente),
- une étape d'optimisation (bloc 29) des spectres précédemment calculés en tenant compte des informations données par le nouveau spectre (pour enlever aux premiers spectres la contribution due au nouveau spectre); en fait, lance un sous-programme 29' qui reteste ici la base de modélisation en supprimant à chaque itération un spectre recalculé (à l'exception des spectres connus de la base de modélisation) et en recherchant un spectre supplémentaire 31 (sous-programme 26) pour remplacer le spectre supprimé 30, avec une incrémentation du spectre recalculé 31 et détection de la condition i > N (bloc 32), c'est-à-dire du moment où tous les spectres recalculés ont été remplacés.

Comme précédemment mentionné, lorsque la base de modélisation contient tous les spectres de modélisation, le spectre de l'échantillon d'effluent de la base se déduit des spectres échantillon par une relation linéaire telle que, par exemple, la relation

$$SPech = C_1\,SP_1 + C_2\,SP_2 + C_3\,SP_3 + C_4\,SP_4 + C_5\,SP_5$$

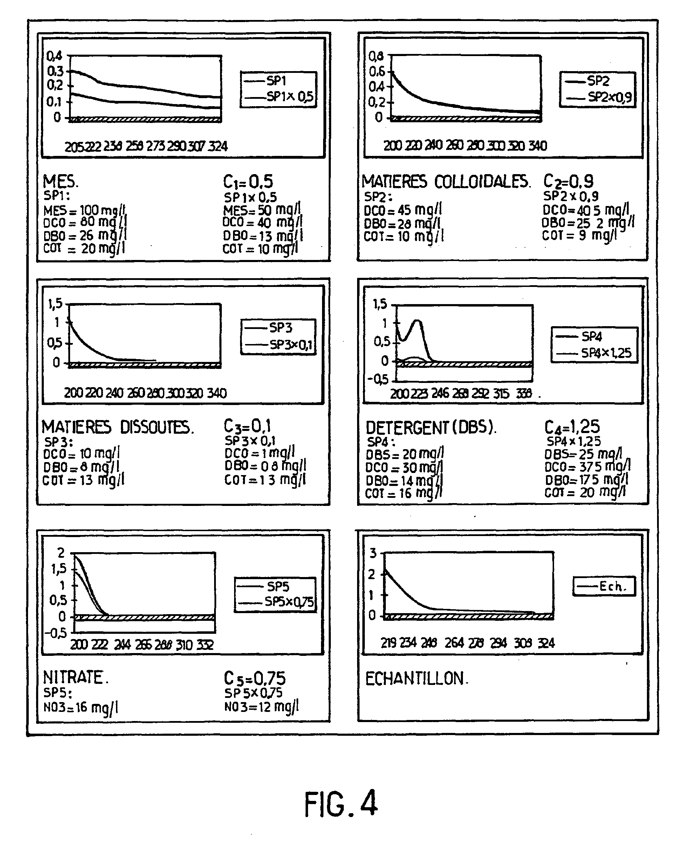

qui correspond à la base de modélisation de la figure 4 qui comprend cinq spectres de modélisation et dans laquelle les coefficients $C_1$ à $C_5$ sont les coefficients de pondération déterminés par déconvolution.

Sur cette figure 4, on a représenté graphiquement les cinq spectres $SP_1$ à $SP_5$ avec en correspondance les valeurs des différents paramètres, pris individuellement et multipliés par le coefficient de pondération, ces paramètres étant :

| | |
|---|---|
| Les matières en suspension | MES |
| La demande en carbone organique | DCO |
| La demande biologique en oxygène | DBO |
| Le carbone organique total | COT |
| Les détergents | DBS |
| Les nitrates | NO3 |

Le Tableau II ci-après permet de fournir les valeurs de ces paramètres en relation avec la formule

$$SPech = (0,5)\,SP_1 + (0,9)\,SP_2 + (0,1)\,SP_3 + (1,25)\,SP_4 + (0,75)\,SP_5$$

**TABLEAU II**

| DCO = 40 + 40,5 + 1 + 37,5 | = 119 mg/l |
|---|---|
| DBO = 13 + 25,2 + 0,8 + 17,5 | = 56,5 mg/l |
| COT = 10 + 9 + 1,3 + 20 | = 40,3 mg/l |
| MES | = 50 mg/l |
| DBS | = 25 mg/l |
| NO3 | =12 mg/l |

**Revendications**

1.  Procédé pour la surveillance dans le temps d'un effluent liquide dont on veut déterminer des paramètres, par exemple des paramètres de pollution, ce procédé comportant une succession de cycles opératoires répétés périodiquement comprenant les étapes suivantes :

    - le prélèvement d'un échantillon de l'effluent liquide que l'on veut surveiller,
    - une analyse spectrophotométrique permettant de relever un spectre de fréquence caractéristique de ce prélèvement,
    - un échantillonnage du spectre obtenu par cette analyse, de manière à obtenir une série d'échantillons du spectre à des fréquences successives,
    - la numérisation de la valeur de ces échantillons de spectre,
    - le stockage en mémoire, avec horodatage, des données numériques relatives à chacun de ces échantillons de spectre et des données correspondant à la fréquence de ces échantillons;

    une phase préalable comportant :

    - l'acquisition de spectres échantillons de référence obtenus par spectrométrie UV d'une pluralité d'échantillons de référence différents, dudit effluant liquide, la mesure de ces spectres échantillon de référence et la détermination en laboratoire des paramètres de ces échantillons de référence, par des méthodes de référence
    - la constitution d'une première bibliothèque incluant des données relatives à ces spectres échantillons
    - l'acquisition et le stockage dans une seconde bibliothèque des données relatives à des spectres d'absorption UV de produis purs connus entrant dans la composition dudit effluent liquide
    - la constitution d'une base de modélisation contenant les données relatives à un nombre minimum N de spectres dont la combinaison linéaire permet d'obtenir un spectre modélisé représentatif du spectre de l'échantillon à analyser, ces données comprenant d'une part un petit nombre M, M < N, de spectres sélectionnés de produits purs connus dont les données sont stockées dans la seconde bibliothèque et un nombre N-M de spectres calculés à partir de spectres sélectionnés et de spectres échantillons;
    - l'obtention d'un modèle de spectre obtenu par combinaison linéaire des spectres de la base de modélisation, et

    une phase opératoire comportant,

    - la comparaison du modèle de spectre et du spectre d'absorption de l'échantillon inconnu à analyser de manière à vérifier la pertinence du modèle.
    - la détermination, par un calcul de déconvolution, des coefficients de pondération des spectres de la base de modélisation dans la quantification du spectre d'absorption de l'échantillon à analyser,
    - la calibration du modèle par le calcul des paramètres de l'effluent liquide en fonction de la valeur des susdits coefficients de pondération,

    la constitution de la base de modélisation étant obtenue à l'issue d'un processus incrémental comprenant les phases suivantes:

- la sélection et l'introduction dans la base de modélisation des données relatives aux M spectres de produits purs connus,
- la sélection d'un spectre échantillon remarquable contenu dans la première bibliothèque en effectuant une déconvolution de chaque spectre échantillon pour connaître la contribution des produits purs connus dans ce spectre échantillon et pour déterminer la fraction résiduelle de ce spectre échantillon par rapport aux spectres connus en choisissant le spectre échantillon remarquable dont la fraction résiduelle est la plus importante,
- l'extraction de la partie du spectre sélectionné qui n'est pas présente dans les spectres déjà rentrés en retranchant au spectre sélectionné une fraction de la somme des spectres déjà rentrés de manière à obtenir un nouveau spectre étalon représentatif de nouveaux produits,
- l'introduction dans la base de modélisation du nouveau spectre étalon,
- le début d'un nouveau cycle de sélection, d'extraction et d'introduction et ce, jusqu'à ce que la base de modélisation soit pleine.

**2.** Procédé selon la revendication 1,
**caractérisé en ce que** la susdite analyse spectrophotométrique comprend une photooxydation d'un échantillon liquide et le relevé du spectre d'absorption UV du prélèvement de l'échantillon liquide photooxydé.

**3.** Procédé selon la revendication 1,
**caractérisé en ce qu'**il comprend en outre la validation de la susdite calibration par l'établissement d'une droite de régression rapprochant les résultats mesurés de ceux estimés par le calcul.

**4.** Procédé selon l'une des revendications précédentes,
**caractérisé en ce que** la susdite base de modélisation est réutilisée pour l'analyse d'échantillons issus de la même source ou de sources similaires.

**5.** Procédé selon la revendication 1,
**caractérisé en ce que**, dans le cas où le liquide à analyser est un effluent industriel, les produits étalon sont des produits purs tels que les nitrates, le nocylène, le tributhylcathechol TBC, des ions nitrites, de l'éthylpropylacrolénie EPA, ainsi que des spectres globaux d'absorption UV d'effluents qualifiés normaux.

**6.** Procédé selon la revendication 1,
**caractérisé en ce qu'**il comprend une phase de vérification de la pertinence du modèle présent dans la base de modélisation, cette vérification consistant à s'assurer que l'écart maximum entre le spectre modélisé et les spectres échantillon demeure inférieur à un seuil prédéterminé.

**7.** Procédé selon l'une des revendications précédentes,
**caractérisé en ce que** le calcul des paramètres de la pollution organique de l'échantillon que l'on veut mesurer comprend un calcul par déconvolution du coefficient de pondération à appliquer à chacun des spectres de la base de modélisation pour obtenir le spectre de cet échantillon et la valeur des paramètres de chacun de ces spectres, puis séparément déterminée à l'aide de valeurs vraies des paramètres de pollution des spectres échantillon.

**8.** Procédé selon la revendication 7,
**caractérisé en ce que** le susdit calcul comprend la réalisation d'une matrice donnant la valeur des paramètres de pollution pour chacun des spectres de la base de modélisation, par un processus de déconvolution, à partir d'au moins une partie des spectres échantillon contenus dans la susdite bibliothèque.

**9.** Dispositif pour la mise en oeuvre du procédé selon l'une des revendications précédentes,
ce dispositif comprenant des moyens (2) permettant d'effectuer une photooxydation de l'échantillon (3), un spectrophotomètre(1) pour l'analyse spectrophotométrique UV de l'échantillon (3) photooxydé et un processeur (9) programmé de manière à effectuer un échantillonnage du spectre obtenu par le spectrophotomètre(1), la numérisation des échantillons ainsi obtenus et la mémorisation, avec horodatage, des valeurs numériques du spectre,
**caractérisé en ce que** le processeur (9) est en outre programmé pour :

- recevoir et stocker en mémoire les données relatives à une pluralité de spectres de produits purs et à une pluralité de spectres échantillon du liquide à analyser,
- constituer une base de modélisation apte à contenir les données numériques d'un nombre N de spectres dont la combinaison linéaire permet d'obtenir un spectre modélisé représentatif du spectre de l'échantillon à analyser,

la constitution de la base de modélisation étant obtenue à l'issue d'un processus incrémental comprenant les phases suivantes :

- la sélection et l'introduction dans la base de modélisation des données relatives aux M spectres de produits purs connus,
- la sélection d'un spectre échantillon remarquable contenu dans la première bibliothèque en effectuant une déconvolution de chaque spectre échantillon pour connaître la contribution des produits purs connus dans ce spectre échantillon et pour déterminer la fraction résiduelle de ce spectre échantillon par rapport aux spectres connus en choisissant le spectre échantillon remarquable dont la fraction résiduelle est la plus importante,
- l'extraction de la partie du spectre sélectionné qui n'est pas présente dans les spectres déjà rentrés en retranchant au spectre sélectionné une fraction de la somme des spectres déjà rentrés de manière à obtenir un nouveau spectre étalon représentatif de nouveaux produits,
- l'introduction dans la base de modélisation du nouveau spectre étalon,
- le début d'un nouveau cycle de sélection, d'extraction et d'introduction et ce, jusqu'à ce que la base de modélisation soit pleine.
- comparer le spectre modélisé au spectre d'absorption de l'échantillon de manière à vérifier la pertinence du modèle,
- déterminer, par un calcul de déconvolution, le coefficient de pondération des spectres de la base de modélisation dans la quantification du spectre d'absorption de l'échantillon à analyser,
- calculer les paramètres de pollution en fonction de la valeur des coefficients de contribution,
- valider cette calibration par l'établissement d'une droite de régression rapprochant les résultats mesurés de ceux obtenus par le calcul.

**10.** Dispositif selon la revendication 9,
**caractérisé en ce que** le dispositif spectrophotométrique (2) comprend une seule source de rayonnement UV permettant à la fois de réaliser un processus de photooxydation et d'assurer la mesure spectrophotométrique.

**11.** Dispositif selon la revendication 10,
**caractérisé en ce qu'**il comporte une cuve d'échantillon (3) comportant deux faces parallèles transparentes (4, 5) traversées par le faisceau de rayonnement UV qui pénètre dans la fente d'un spectrophotomètre UV.

**12.** Dispositif selon la revendication 11,
**caractérisé en ce que** la source lumineuse (2) est accolée à l'une desdites faces (4),

## Claims

**1.** A method for monitoring over time a liquid effluent, the parameters of which are intended to be determined, for example pollution parameters, this method including a succession of periodically repeated operating cycles, comprising the following steps:

- taking a sample from the liquid effluent intended to be monitored,
- performing a spectrophotometric analysis in order to record a frequency spectrum characteristic of this sample,
- sampling the spectrum obtained by this analysis, so as to obtain a series of spectrum samples at successive frequencies,
- digitizing the value of these spectrum samples,
- storing in memory with time stamping, digital data relative to each of these spectrum samples and data corresponding to the frequency of these samples;

a preliminary phase including:

- acquiring sample reference spectra obtained by UV spectrometry of a plurality of different reference samples of said liquid effluent, measuring these sample reference spectra and determining in the laboratory, parameters of these reference samples by reference methods
- forming a first library including data relative to these sample spectra
- acquiring and storing in a second library, data relative to UV absorption spectra of known pure products being part of the composition of said liquid effluent
- forming a modeling base containing data relative to a minimum number N of spectra, with the linear combination of which a modeled spectrum representative of the spectrum of the sample to be analyzed may be obtained, these data comprising a small number M, M < N, of selected spectra of known pure products, the data of which are stored in the second library on the one hand and a number N-M of spectra calculated from selected spectra and sample spectra on the other hand;
- obtaining a spectrum model obtained by linear combination of the spectra of the modeling base, and

an operating phase including,

- comparing the spectrum model and the absorption spectrum of the unknown sample to be analyzed in order to verify the relevance of the model,
- determining by a deconvolution calculation, the weighting coefficients of the spectra of the modeling base in the quantification of the absorption spectrum of the sample to be analyzed,
- calibrating the model by calculating parameters of the liquid effluent depending on the value of the aforesaid weighting coefficients,

the formation of the modeling base being obtained upon completion of an incremental process comprising the following phases:

- selecting and introducing into the modeling base, data relative to M spectra of known pure products,
- selecting a remarkable sample spectrum contained in the first library by carrying out deconvolution of each sample spectrum in order to be acquainted with the contribution of the known pure products in this sample spectrum and in order to determine the residual fraction of this sample spectrum relatively to the known spectra by selecting the remarkable sample spectrum, for which the residual fraction is the most significant,
- extracting the portion of the selected spectrum which is not present in the already entered spectra by subtracting from the selected spectrum a fraction of the sum of the already entered spectra so as to obtain a new spectrum standard representative of new products,
- introducing the new spectrum standard into the modeling base,
- beginning a new selection, extraction and introduction cycle and this until the modeling base is full.

2. The method according to claim 1,
**characterized in that** the aforesaid spectrophotometric analysis comprises photo-oxidation of a liquid sample and the plotting of the UV absorption spectrum of the taken amount of photo-oxidized liquid sample.

3. The method according to claim 1,
**characterized in that** it further comprises validation of the aforesaid calibration by establishing a regression line to bring together the measured results and those estimated by the calculation.

4. The method according to any of the preceding claims,
**characterized in that** the aforesaid modeling base is reused for analyzing samples from the same source or from similar sources.

5. The method according to claim 1,
**characterized in that**, in the case when the liquid to be analyzed is an industrial effluent, the standard products are pure products, such as nitrates, nocylene, tributyl cathechol TBC, nitrite ions, ethylpropylacrolein EPA, as well as global UV absorption spectra of so-called normal effluents.

6. The method according to claim 1
**characterized in that** it comprises a phase for verifying the relevance of the model present in the modeling base, this verification consisting of making sure that the maximum deviation between the modeled spectrum and the sample spectra remains below a predetermined threshold.

7. The method according to any of the preceding claims,
**characterized in that** the calculation of the parameters of the organic pollution of the sample which is intended to be measured, comprises a calculation by deconvolution of the weighting coefficient to be applied to each of the spectra of the modeling base in order to obtain the spectrum of this sample and the value of the parameters of each of these spectra, and then separately determined by means of the real values of the pollution parameters of these sample spectra.

**8.** The method according to claim 7,
**characterized in that** the aforesaid calculation comprises constructing a matrix giving the value of the pollution parameters for each of the spectra of the modeling base, by means of a deconvolution process, from at least one portion of the sample spectra contained in the aforesaid library.

**9.** A device for applying the method according to any of the preceding claims,
this device comprising means (2) for carrying out photo-oxidation of the sample (3), a spectrophotometer (1) for UV spectrophotometric analysis of the photo-oxidized sample (3) and a processor (9) programmed so as to carry out sampling of the spectrum obtained by the spectrophotometer (1), digitization of the thereby obtained samples and storage in memory with time stamping of the digital values of the spectrum,
**characterized in that** the processor (9) is further programmed for:

- receiving and storing in memory the data relative to a plurality of spectra of pure products and to a plurality of sample spectra of the liquid to be analyzed,
- forming a modeling base capable of containing the digital data of a number N of spectra, with the linear combination of which a modeled spectrum representative of the spectrum of the sample to be analyzed may be obtained,

the formation of the modeling base being obtained upon completion of an incremental process comprising the following phases:

- selecting and introducing into the modeling base, data relative to the M spectra of known pure products,
- selecting a remarkable sample spectrum contained in the first library by carrying out a deconvolution of each sample spectrum in order to be acquainted with the contribution of the known pure products in this sample spectrum and to determine the residual fraction of this sample spectrum relatively to the known spectra by selecting the remarkable sample spectrum, the residual fraction of which is the most significant,
- extracting the portion of the selected spectrum which is not present in the already entered spectra by subtracting from the selected spectrum a fraction of the sum of the already entered spectra in order to obtain a new spectrum standard representative of new products,
- introducing the new spectrum standard into the modeling base,
- beginning a new selection, extraction and introduction cycle, and this until the modeling base is full,
- comparing the modeled spectrum with the absorption spectrum of the sample in order to verify relevance of the model,
- determining by a deconvolution calculation, the weighting coefficient of the spectra of the modeling base in the quantification of the absorption spectrum of the sample to be analyzed,
- calculating the pollution parameters depending on the value of the contribution coefficients,
- validating this calibration by establishing a regression line to bring together the measured results and those obtained by the calculation.

**10.** The device according to claim 9,
**characterized in that** the spectrophotometric device (2) comprises a single source of UV radiation with which it is possible to both achieve a photo-oxidization process and provide the spectrophotometric measurement.

**11.** The device according to claim 10,
**characterized in that** it includes a sample tank (3) including two transparent parallel faces (4, 5) crossed by the UV radiation beam which penetrates into the slit of an UV spectrophotometer.

**12.** The device according to claim 11,
**characterized in that** the light source (12) is placed adjacent to one of said faces (4).


## Patentansprüche

**1.** Verfahren zur Zeitüberwachung von anfallendem Flüssigabfall, wobei man Parameter bestimmen will, zum Beispiel Verschmutzungsparameter, wobei dieses Verfahren eine Folge von sich zeitweise wiederholenden Ausführungszyklen umfasst, die folgende Etappen umfassen:

- die Probeentnahme des zu überwachenden Flüssigabfalls,

- eine spektrofotometrische Analyse, wodurch es ermöglicht ist, ein charakteristisches Frequenzspektrum von dieser Probeentnahme festzustellen,

- eine Probeentnahme des durch diese Analyse erhaltenen Spektrums, um eine Reihe von Proben des Spektrums mit aufeinander folgenden Frequenzen zu erhalten,

- die Digitalisierung des Wertes dieser Spektrenproben,

- die Speicherung im Speicher, mit Zeitstempel, der numerischen Daten, die sich auf jede dieser Spektrenproben und auf die der Frequenz dieser Proben entsprechenden Daten beziehen;

eine Vorphase, folgendes umfassend:

- die Erfassung von Spektren der Referenzproben, die durch eine UV-Spektrometrie von zahlreichen verschiedenen Referenzproben des genannten Flüssigabfalls, von der Messung dieser Spektren von Referenzproben und der Ermittlung im Labor der Parameter dieser Referenzproben durch Referenzmethoden erhalten wurden

- die Bildung von einer ersten Bibliothek, die Daten umfaßt, die sich auf diese Spektren von Proben beziehen

- die Erfassung und Speicherung in einer zweiten Bibliothek von Daten, die sich auf die Spektren von einer UV-Absorbierung von bekannten reinen Produkten beziehen, die in der Zusammensetzung des genannten Flüssigabfalls hineinpassen

- die Bildung von einer Modellierungsgrundlage, die die Daten umfasst, die sich auf eine Mindestzahl N von Spektren bezieht, deren lineare Kombination es gestattet, ein repräsentatives modelliertes Spektrum für das Spektrum der zu analysierenden Probe zu erhalten, wobei diese Daten einerseits eine kleine Zahl M, M < N von Spektren umfassen, die ausgewählt sind aus bekannten reinen Produkten, wobei die Daten in der zweiten Bibliothek gespeichert werden und eine Zahl N-M von Spektren werden aus den ausgewählten Spektren und aus den Spektren von Proben berechnet,

- der Erhalt von einem Spektrenmodell, das durch eine lineare Kombination der Spektren der Modellierungsgrundlage erhalten wurde, und

eine Vorgehensphase, die folgendes umfasst:

- den Vergleich des Spektrenmodells und des Absorptionsspektrums der zu analysierenden unbekannten Probe, um die Sachdienlichkeit des Modells zu überprüfen.

- die Ermittlung durch eine Dekonvolutionsberechnung der Bewertungskoeffizienten der Spektren der Modellierungsgrundlage in der Quantifizierung des Absorptionsspektrums der zu analysierenden Probe,

- die Kalibrierung des Modells durch die Berechnung der Parameter des Flüssigabfalls gemäß dem Wert der besagten Bewertungskoeffizienten,

die Bildung der Modellierungsgrundlage, die am Ende von einem inkrementalen Prozess erhalten wurde, der folgende Phasen umfasst;

- die Selektion und die Einführung in die Modellierungsgrundlage der Daten, die sich auf die M-Spektren von bekannten reinen Produkten beziehen,

- die Selektion von einer beachtenswerten Spektrenprobe, die in der ersten Bibliothek enthalten ist, indem eine Dekonvolution von einer jeden Spektrenprobe ausgeführt wird, um den Beitrag der bekannten reinen Produkte in dieser Spektrenprobe zu kennen und um den restlichen Bruchteil dieser Spektrenprobe im Vergleich zu den bekannten Spektren zu bestimmen, indem man die beachtenswerte Spektrenprobe wählt, deren restlicher Bruchteil der wichtigste ist,

- die Extraktion des Teils des ausgewählten Spektrums, der nicht in den bereits eingeholten Spektren vorliegt, indem man von dem ausgewählten Spektrum einen Bruchteil der Summe von bereits eingebrachten Spektren abzieht, um ein neues repräsentatives Richtspektrum für neue Produkte zu erhalten,

- die Einführung in die Modellierungsgrundlage des neuen Richtspektrums,

- der Anfang von einem neuen Selektions-, Extraktion- und Einführungszyklus und dieses, bis die Modellierungsgrundlage vollständig ist.

2. Verfahren nach Anspruch 1, **gekennzeichnet dadurch, dass** die besagte spektrofotometrische Analyse eine Photooxydation von einer flüssigen Probe und die Ermittlung des UV-Absorptionsspektrums der photooxydierten flüssigen Probeentnahme umfasst.

3. Verfahren nach Anspruch 1, **gekennzeichnet dadurch, dass** es außerdem die Validierung der besagten Kalibrierung umfasst durch die Erstellung von einer Regressionsgeraden, indem die gemessenen Ergebnisse jenen angenähert werden, die durch die Berechnung geschätzt wurden.

4. Verfahren nach einem der vorangehenden Ansprüche, **gekennzeichnet dadurch, dass** die besagte Modellierungsgrundlage für die Analyse von Proben, die aus der gleichen Quelle oder aus ähnlichen Quellen stammen, wieder verwendet wurde.

5. Verfahren nach Anspruch 1, **gekennzeichnet dadurch, dass** im Falle, wo die zu analysierende Flüssigkeit ein industrielles Abwasser ist, die Richtprodukte reine Produkte sind, wie Nitrate, Nocylene, Tributhylcathechol TBC, Nitrit-Ionen, Ethylpropylacrolein EPA sowie globale UV-Absorbierungsspektren von für normal qualifizierte Abwässer.

6. Verfahren nach Anspruch 1, **gekennzeichnet dadurch, dass** es eine Überprüfungsphase der Sachdienlichkeit des vorliegenden Modells in der Modellierungsgrundlage umfasst, wobei diese Überprüfung darin besteht, sich zu vergewissern, dass der Höchstabstand zwischen dem modellierten Spektrum und den Probenspektren unter einer vorgegebenen Schwelle bleibt.

7. Verfahren nach einem der vorangehenden Ansprüche, **gekennzeichnet dadurch, dass** die Berechnung der Parameter der organischen Verschmutzung der zu messenden Probe eine Berechnung durch Dekonvolution des Bewertungskoeffizienten umfasst, die an jedem der Spektren der Modellierungsgrundlage anzuwenden ist, um das Spektrum von dieser Probe und den Wert der Parameter von jeden diesen Spektren zu erhalten, das heißt gesondert bestimmt mit Hilfe von wahren Werten der Verschmutzungsparameter der Spektren von Proben.

8. Verfahren nach Anspruch 7, **gekennzeichnet dadurch, dass** die besagte Berechnung die Verwirklichung von einer Matrix umfasst, die den Wert der Parameter der Verschmutzung für jedes der Spektren der Modellierungsgrundlage ergibt, durch einen Dekonvolutionsprozess, ab mindestens einem Teil der Probenspektren, der in der besagten Bibliothek enthalten ist.

9. Vorrichtung zur Ausführung des Verfahrens nach einem der vorangehenden Ansprüche, wobei diese Vorrichtung Mittel (2) umfasst, wodurch es ermöglicht ist, eine Fotooxydation der Probe (3) durchzuführen, einen Spektrophotometer (1) für die spektrofotometrische UV-Analyse der photooxydierten Probe (3) und einen programmierten Prozessor (9), um eine durch das Spektrofotometer (1) erhaltene Probeentnahme des Spektrums durchzuführen, die Digitalisierung der auf diese Weise erhaltenen Proben und die Speicherung mit Zeitstempel, digitale Werte des Spektrums, **gekennzeichnet dadurch, dass** der Prozessor (9) außerdem programmiert ist, um:

- die auf eine Vielzahl von Spektren von reinen Produkten und die auf eine Vielzahl von Spektren von Proben der zu analysierenden Flüssigkeit bezogenen Daten zu empfangen und im Speicher zu speichern,
- eine Modellierungsgrundlage zu bilden, die dazu geeignet ist, die numerischen Daten von einer Zahl N von Spektren zu enthalten, wobei es die lineare Kombination gestattet, ein repräsentatives modelliertes Spektrum für das Spektrum der zu analysierenden Probe zu erhalten,

die Bildung der Modellierungsgrundlage wird am Ende von einem inkrementalen Prozess erhalten, der folgende Phasen umfasst:

- die Selektion und die Einführung in die Modellierungsgrundlage von Daten bezüglich der M-Spektren von bekannten reinen Produkten,
- die Selektion eines Spektrums von einer beachtenswerten Probe, die in der ersten Bibliothek enthalten ist, indem eine Dekonvolution eines jeden Probenspektrums ausgeführt wird, um den Beitrag der bekannten reinen Produkte in diesem Spektrum von einer Probe zu kennen und um das restliche Bruchteil von diesem Probenspektrum im Vergleich zu den bekannten Spektren zu bestimmen, indem man das beachtenswerte Probenspektrum wählt, dessen restliches Bruchteil das wichtigste ist,
- die Extraktion des Teils des ausgewählten Spektrums, das nicht in den bereits eingebrachten Spektren vorliegt, indem man von dem ausgewählten Spektrum einen Bruchteil der Summe der bereits eingebrachten Spektren abzieht, um ein neues repräsentatives Richtspektrum für neue Produkte zu erhalten.
- die Einführung des neuen Richtspektrums in die Modellierungsgrundlage
- der Beginn eines neuen Selektions-, Extraktions- und Einführungszyklus und dieses bis zur Vervollständigung der Modellierungsgrundlage.
- das modellierte Spektrum mit dem Absorptionsspektrum der Probe vergleichen, um die Sachdienlichkeit des Modells zu überprüfen,

- Bestimmung, durch eine Dekonvolutionsberechnung, des Bewertungskoeffizienten der Spektren der Modellierungsgrundlage in der Quantifizierung des Absorptionsspektrums der zu analysierenden Probe,
- Berechnung der Verschmutzungsparameter gemäß dem Wert der Beitragskoeffizienten,
- Validierung dieser Kalibrierung durch die Erstellung von einer Regressionsgeraden, indem die gemessenen Ergebnisse denen durch Berechnung erhaltenen Ergebnisse angenähert werden

10. Vorrichtung nach Anspruch 9, **gekennzeichnet dadurch, dass** die spektrofotometrische Vorrichtung (2) eine einzige Quelle von UV-Strahlen umfasst, wodurch es ermöglich ist, gleichzeitig einen Fotooxydationsprozess zu verwirklichen und das spektrofotometrische Maß zu sichern.

11. Vorrichtung nach Anspruch 10, **gekennzeichnet dadurch, dass** es ein Probegefäß (3) umfasst mit zwei durchsichtigen parallelen Seiten (4, 5), die von dem UV-Strahlenbündel durchstrahlt werden, das in den Spalt eines UV-Spektrofotometers eindringt.

12. Vorrichtung nach Anspruch 11, **gekennzeichnet dadurch, dass** die Lichtquelle (2) an eine der besagten Seiten (4) angefügt ist.

FIG.2

FIG.1

BOUCLE PRINCIPALE.

Initialisation de la base du modèle avec les Nc spectres connus. — 20

19

Incrémentation de l'ordre de la base. — 21

Ordre de la base >=8 ou pertinence de la base suffisante? — 22

Non      Oui

FIN — 23

RECHERCHE D'UN SPECTRE SUPPLEMENTAIRE.

26

Cherche le spectre i le plus éloigné de la base. — 27

Ajoute à la base le spectre déduit du spectre i. — 28

RETOUR

INCREMENTATION DE L'ORDRE DE LA BASE.

Recherche d'un spectre supplémentaire. — 24 — 25

Optimisation des spectres précédemment calculés. — 29

RETOUR

OPTIMISATION DES SPECTRES DE LA BASE (AVEC Nc SPECTRES CONNUS et N SPECTRES RECALCULES.)

29'

i = 1

Suppression du spectre recalculé i. — 30

Recherche d'un spectre supplémentaire pour remplacer le spectre supprimé. — 31

i = i + 1 — 31'

Non     i > N     Oui — 32

RETOUR

FIG.3

FIG. 4

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 0682250 A
- FR 2783322
- FR 2787883

**Littérature non-brevet citée dans la description**

- **O. THOMAS et al.** Advanced UV examination of wastewater. *ENVIRONMENTAL TECHNOLOGY,* 1996, vol. 17, 251-261